# EUROPEAN PATENT SPECIFICATION

(11) **EP 1 140 044 B1**
(45) Date of publication and mention of the grant of the patent: **15.03.2006**
(21) Application number: 99964644.1
(22) Date of filing: 21.12.1999
(51) Int. Cl.: A61K 31/00, A61K 31/517, A61K 31/519, A61K 9/72, A61P 15/00, A61P 15/10

(54) **cGMP PDE 5 INHIBITORS FOR INHALATION IN THE TREATMENT OF SEXUAL DYSFUNCTION**
CGMP PDE5 INHIBITOREN ZUR INHALATIONSBEHANDLUNG VON SEXUELLER DYSFUNKTION
INHIBITEURS DE L'ACIDE GUANOSINE-3'5'-MONOPHOSPHATE CYCLIQUE PHOSPHODIESTERASES 5 SOUS FORME INHALABLE PERMETTANT DE TRAITER LES TROUBLES SEXUELS

(30) Priority: 22.12.1998 GB 9828340
(43) Date of publication of application: 10.10.2001
(73) Proprietor: Novartis AG, 4056 Basel (CH); Novartis Pharma GmbH, 1230 Wien (AT)
(72) Inventor: NAEF, Reto, CH-4310 Rheinfelden (CH)
(74) Representative: de Weerd, Petrus G.W.
(86) International application number: PCT/EP1999/010250
(87) International publication number: WO 2000/037061

(56) References cited:
- EP-A- 0 636 626
- EP-A- 0 771 799
- WO-A-94/28902
- WO-A-95/09636
- WO-A-96/16644
- WO-A-96/28448
- WO-A-99/64004
- DE-A- 4 420 708
- US-A- 5 436 233
- US-A- 6 018 046
- ICHINOSE ET AL.: "Selective pulmonary vasodilation induced by aerosolized Zaprinast" ANESTHESIOLOGY, vol. 88, no. 2, February 1998 (1998-02), pages 410-416, XP000913484
- FUJIMURA ET AL.: "Bronchoprotective effect of an intrabronchial administration of cilostazol powder and nebulized PDE1 and PDE4 inhibitor KF19514 in guinea pigs" INT. ARCH. ALLERGY IMMUNOL., vol. 116, no. 3, July 1998 (1998-07), pages 220-227, XP000913884

## Description

This invention relates to the use of organic compounds, in particular certain inhibitors of cyclic guanosine 3',5'-monophosphate phosphodiesterases (cGMP PDEs), in inhalable form for the preparation of a medicament for the treatment of sexual dysfunction, including male erectile dysfunction and female sexual dysfunction.

International patent applications WO 94/28902, WO 96/16644 and WO 96/16657 describe the use of selective inhibitors of cyclic guanosine 3',5'-monophosphate phosphodiesterases (cGMP PDEs), particularly cGMP PDE 5 inhibitors, in the treatment of sexual dysfunction. These references state that, for human use, the inhibitors are administered orally or, where the recipient suffers from a swallowing disorder or from impairment of drug absorption after oral administration, parenterally, sublingual and buccal administration being suggested as means of parenteral administration.

United States patent specification US 5436233 discloses 4-aminoquinazoline derivatives and their use as cGMP PDE inhibitors or additionally TXA₂ synthetase inhibitors for treating various conditions.

European patent specification EP 636626 discloses pyrazolopyrimidine derivatives and their use as cGMP PDE inhibitors for treating cardiovascular disorders.

United States patent specification US 5294612 discloses 6-heterocyclyl-pyrazolo[3,4-d]pyrimidin-4-ones and their use as PDE inhibitors for treating cardiovascular diseases.

It has now surprisingly been found that certain cGMP PDE inhibitors including compounds effective in the treatment, i.e. curative or prophylactic treatment, of sexual dysfunction exhibit maximum concentration in plasma in a very short time following administration by inhalation, i.e. by delivery to the lung, indicating a more rapid onset of action for the compounds when administered by this route.

Accordingly, the present invention provides, in one aspect, the use of a cGMP PDE 5 inhibitor in inhalable form for the preparation of an inhalable medicament for the treatment of sexual dysfunction, in which said inhibitor is 5-[2-ethoxy-5-(4-methylpiperazinyl-sulfonyl)phenyl]-1-methyl-3-n-propyl-1,6-dihydro-7H-pyrazolo[4,3-d]-pyrimidin-7-one, 4-phenylmethylamino-6-chloro-2-(1-imidazolyl)-quinazoline, 4-phenyl-methylamino-6-chloro-2-(3-pyridyl)-quinazoline, 1,3-dimethyl-6-(2-propoxy-5-methane-sulfonylamidophenyl)-1,5-dihydro-pyrazolo[3,4-d]pyrimidin-4-one or 1-cyclopentyl-3-ethyl-6-(3-ethoxy-4-pyridyl)-pyrazolo[3,4-d]pyrimidin-4-one.
5-[2-Ethoxy-5-(4-methylpiperazinyl-sulfonyl)phenyl]-1-methyl-3-n-propyl-1,6-dihydro-7H-pyrazolo[4,3-d]-pyrimidin-7-one is disclosed in WO 94/28902.
4-Phenylmethylamino-6-chloro-2-(1-imidazolyl)quinazoline and 4-phenyl-methylamino-6-chloro-2-(3-pyridyl)-quinazoline are disclosed in US 5436233.
1,3-Dimethyl-6-(2-propoxy-5-methane-sulfonylamidophenyl)-1,5-dihydro-pyrazolo[3,4-d]pyrimidin-4-one is disclosed in EP 636626.
1-Cyclopentyl-3-ethyl-6-(3-ethoxy-4-pyridyl)-pyrazolo[3,4-d]pyrimidin-4-one is disclosed US 5294612.

The inhalable form of the aforementioned cGMP PDE 5 inhibitors, i.e. the form suitable for delivery to the lung, may be, for example, an atomisable composition such as an aerosol comprising the active ingredient in solution or dispersion in a propellant or a nebulizable composition comprising a dispersion of the active ingredient in an aqueous, organic or aqueous/organic medium, or a finely divided particulate form comprising the active ingredient in finely divided form optionally together with a pharmaceutically acceptable carrier in finely divided form.

An aerosol composition suitable for use as the inhalable form of the medicament may comprise one of the cGMP PDE 5 inhibitors as the active ingredient in solution or dispersion in a propellant, which may be chosen from any of the propellants known in the art. Suitable such propellants include hydrocarbons such as n-propane, n-butane or isobutane or mixtures of two or more such hydrocarbons, and halogen-substituted hydrocarbons, for example fluorine-substituted methanes, ethanes, propanes, butanes, cyclopropanes or cyclobutanes, particularly 1,1,1,2-tetrafluoroethane (HFA134a) and heptafluoropropane (HFA227), or mixtures of two or more such halogen-substituted hydrocarbons. Where the active ingredient is present in dispersion in the propellant, i.e. where it is present in particulate form dispersed in the propellant, the aerosol composition may also contain a lubricant and a surfactant, which may be chosen from those lubricants and surfactants known in the art. The aerosol composition may contain up to about 5% by weight, for example 0.002 to 5%, 0.01 to 3%, 0.015 to 2%, 0.1 to 2%, 0.5 to 2% or 0.5 to 1%, by weight of the active ingredient, based on the weight of the propellant. Where present, the lubricant and surfactant may be in an amount up to 5% and 0.5% respectively by weight of the aerosol composition. The aerosol composition may also contain ethanol as co-solvent in an amount up to 30% by weight of the composition, particularly for administration from a pressurised metered dose inhalation device.

A finely divided particulate form, i.e. a dry powder, suitable for use as the inhalable form of the medicament may comprise the active ingredient in finely divided particulate form, optionally together with a particulate carrier, which may be chosen from materials known as carriers in dry powder inhalation compositions, for example saccharides, including monosaccharides, disaccharides and polysaccharides such as arabinose, glucose, fructose, ribose, mannose, sucrose, lactose, maltose, starches or dextran and sugar alcohols such as mannitol. An especially preferred carrier is lactose. The carrier may be finely divided, e.g. finely divided but of larger particle size than the active ingredient, or may comprise a mixture of particles of similar size to the active ingredient and larger particles. The dry powder may be in capsules of gelatin or plastic, or in blisters, for use in a dry powder inhalation device, preferably in dosage units of 5 mg to 40 mg of the active ingredient. Alternatively, the dry powder may be contained in a reservoir in a multi-dose dry powder inhalation device.

In the finely divided particulate form of the medicament, and in the aerosol composition where the active ingredient is present in particulate form, the active ingredient may have an average particle diameter of up to about 10 µm, for example 0.1 to 5 µm, preferably 1 to 5 µm. The particle size of the active ingredient, and that of a solid carrier where present in dry powder compositions, can be reduced to the desired level by conventional methods, for example by grinding in an air-jet mill, ball mill or vibrator mill, microprecipitation, spray-drying, lyophilisation or recrystallisation from supercritical media.

The inhalable medicament may be administered using an inhalation device suitable for the inhalable form, such devices being well known in the art. Accordingly, the invention also provides a pharmaceutical product comprising one of the cGMP PDE 5 inhibitors in inhalable form as hereinbefore described in association with an inhalation device. In a further aspect, the invention provides an inhalation device containing one of the cGMP PDE 5 inhibitors in inhalable form as hereinbefore described.

Where the inhalable form of the active ingredient is an aerosol composition, the inhalation device may be an aerosol vial provided with a valve adapted to deliver a metered dose, such as 10 to 100 µl, e.g. 25 to 50 µl, of the composition, i.e. a device known as a metered dose inhaler. Suitable such aerosol vials and procedures for containing within them aerosol compositions under pressure are well known to those skilled in the art of inhalation therapy. Where the inhalable form of the active ingredient is a nebulizable aqueous, organic or aqueous/organic dispersion, the inhalation device may be a known nebulizer, for example a conventional pneumatic nebulizer such as an airjet nebulizer, or an ultrasonic nebulizer, which may contain, for example, from 1 to 50 ml, commonly 1 to 10 ml, of the dispersion; or a hand-held nebulizer such as an AERx (ex Aradigm, US) or BINEB (Boehringer Ingelheimy nebulizer which allows much smaller nebulized volumes, e.g. 10 to 100 µl, than conventional nebulizers. Where the inhalable form of the active ingredient is the finely divided particulate form, the inhalation device may be, for example, a dry powder inhalation device adapted to deliver dry powder from a capsule or blister containing a dosage unit of the dry powder, for example a dry powder capsule inhaler as described in US3991761, which may be modified by coating the capsule-piercing pins with a polymer as described in WO99/45987, or a multidose dry powder inhalation device adapted to deliver, for example, 5 mg to 25 mg of dry powder per actuation. Suitable such dry powder inhalation devices are well known, for example the device described in WO97/20589.

The invention is illustrated by the following Examples.

### Example I

Gelatin capsules suitable for use in a capsule inhaler are prepared, each capsule containing a dry powder consisting of 10 mg of 5-[2-ethoxy-5-(4-methylpiperazinylsulfonyl)phenyl]-1-methyl-3-n-propyl-1,6-dihydro-7H-pyrazolo[4,3-d]pyrimidin-7-one (hereinafter Compound A) which has been ground to a mean particle diameter of 1-5 µm in an air jet mill and 10 mg of lactose monohydrate having a particle diameter below 212 µm. These capsules are used in the treatment of erectile dysfunction patients by inserting a capsule into the capsule chamber of the inhaler described in US3991761 and actuating the piercing devices to perforate the capsule and release the dry powder when air is inhaled through the capsule chamber by a patient.

### Examples 2 - 34

Example 1 is repeated using the amounts of Compound A and lactose monohydrate shown below instead of the amounts used in Example 1:

| Example | Amount of Compound A | Amount of Lactose Monohydrate |
|---|---|---|
| 2 | 10 mg | 15 mg |
| 3 | 10 mg | 20 mg |
| 4 | 10 mg | 25 mg |
| 5 | 10 mg | 30 mg |
| 6 | 10 mg | 35 mg |
| 7 | 10 mg | 40 mg |
| 8 | 15 mg | 5 mg |
| 9 | 15 mg | 10 mg |
| 10 | 15 mg | 15 mg |
| 11 | 15 mg | 20 mg |
| 12 | 15 mg | 25 mg |
| 13 | 15 mg | 30 mg |
| 14 | 15 mg | 35 mg |
| 15 | 20 mg | 5 mg |
| 16 | 20 mg | 10 mg |
| 17 | 20 mg | 15 mg |
| 18 | 20 mg | 20 mg |
| 19 | 20 mg | 25 mg |
| 20 | 20 mg | 30 mg |
| 21 | 25 mg | 5 mg |
| 22 | 25 mg | 10 mg |
| 23 | 25 mg | 15 mg- |
| 24 | 25 mg | 20 mg |
| 25 | 25 mg | 25 mg |
| 26 | 30 mg | 5 mg |
| 27 | 30 mg | 10 mg |
| 28 | 30 mg | 15 mg |
| 29 | 30 mg | 20 mg |
| 30 | 35 mg | 5 mg |
| 31 | 35 mg | 10 mg |
| 32 | 35 mg | 15 mg |
| 33 | 40 mg | 5 mg |
| 34 | 40 mg | 10 mg |

### Examples 35 - 54

Example 1 is repeated, but using 4- phenylmethylamino-6-chloro-2-(1-imidazolyl)quinazoline (hereinafter Compound B) as the active ingredient instead of Compound A used in that example and using amounts of active ingredient and lactose monohydrate as shown below:

| Example | Amount of Compound B | Amount of Lactose Monohydrate |
|---|---|---|
| 35 | 10 mg | 5 mg |
| 36 | 10 mg | 10 mg |
| 37 | 10 mg | 15 mg |
| 38 | 10 mg | 20 mg |
| 39 | 10 mg | 25 mg |
| 40 | 10 mg | 30 mg |
| 41 | 10 mg | 35 mg |
| 42 | 10 mg | 40 mg |
| 43 | 20 mg | 5 mg |
| 44 | 20 mg | 10 mg |
| 45 | 20 mg | 15 mg |
| 46 | 20 mg | 20 mg |
| 47 | 20 mg | 25 mg |
| 48 | 20 mg | 30 mg |
| 49 | 30 mg | 5 mg |
| 50 | 30 mg | 10 mg |
| 51 | 30 mg | 15 mg |
| 52 | 30 mg | 20 mg |
| 53 | 40 mg | 5 mg |
| 54 | 40 mg | 10 mg |

### Examples 55 - 69

Example 1 is repeated, but using 1-cyclopentyl-3-ethyl-6-(3-ethoxy-4-pyridyl)-pyrazolo[3,4-d]pyrimidin-4-one (hereinafter referred to alternatively as Compound C) as the active ingredient instead of Compound A used in that Example and using amounts of active ingredient and lactose monohydrate as shown below:

| Example | Amount of Compound C | Amount of Lactose Monohydrate |
|---|---|---|
| 55 | 10 mg | 5 mg |
| 56 | 10 mg | 10 mg |
| 57 | 10 mg | 15 mg |
| 58 | 10 mg | 20 mg |
| 59 | 10 mg | 25 mg |
| 60 | 10 mg | 30 mg |
| 61 | 10 mg | 40 mg |
| 62 | 20 mg | 5 mg |
| 63 | 20 mg | 10 mg |
| 64 | 20 mg | 15 mg |
| 65 | 20 mg | 20 mg |
| 66 | 20 mg | 30 mg |
| 67 | 30 mg | 5 mg |
| 68 | 30 mg | 10 mg |
| 69 | 30 mg | 20 mg |

### Examples 70 - 82

Example - 1 is repeated, but using 1,3-dimethyl-6-(-2-propoxy-5-methanesulfonylamidophenyl)-1,5-dihydropyrazolo[3,4-d]pyrimidin-4-one (hereinafter referred to as Compound D) as active ingredient instead of Compound A used in that Example, and using amounts of compound D and lactose monohydrate as shown below:

| Example | Amount of Compound D | Amount of Lactose Monohydrate |
|---|---|---|
| 70 | 10 mg | 10 mg |
| 71 | 10 mg | 15 mg |
| 72 | 10 mg | 20 mg |
| 73 | 10 mg | 30 mg |
| 74 | 10 mg | 40 mg |
| 75 | 20 mg | 5 mg |
| 76 | 20 mg | 10 mg |
| 77 | 20 mg | 15 mg |
| 78 | 20 mg | 20 mg |
| 79 | 20 mg | 30 mg |
| 80 | 30 mg | 5 mg |
| 81 | 30mg | 10 mg |
| 82 | 30 mg | 20 mg |

### Examples 83 - 92

Example 1 is repeated, but using 4-phenylmethylamino-6-chloro-2-(3-pyridyl)quinazoline (hereinafter referred to alternatively as Compound E) as active ingredient instead of Compound A used in that example, and using amount of Compound E and lactose monohydrate as shown below:

| Example | Amount of Compound D | Amount of Lactose Monohydrate |
|---|---|---|
| 83 | 10 mg | 10 mg |
| 84 | 10 mg | 15 mg |
| 85 | 10 mg | 20 mg |
| 86 | 10 mg | 30 mg |
| 87 | 20 mg | 5 mg |
| 88 | 20 mg | 10 mg |
| 89 | 20 mg | 15 mg |
| 90 | 20 mg | 20 mg |
| 91 | 30 mg | 5 mg |
| 92 | 30 mg | 10 mg |

### Example 93

The absorption time for 5-[2-ethoxy-5-(4-methylpiperazinylsuifonyl)phenyl]-1-methyl-3-n-propyl-1,6-dihydro-7H-pyrazolo[4,3-d]pyrimidin-7-one, hereinafter Compound A, following administration by inhalation is measured in a rodent pharmacokinetic model which simulates inhalation as a route of administration. Male Wistar:Han rats used for this model are supplied by Harlan UK Ltd at 105 days of age and approximately 350g weight. They are fed ad libitum with a standard laboratory diet and housed under controlled conditions (12 hours light/dark cycle; 20°C) for at least one week prior to the experiment.

Compound A is dissolved, at a concentration of 1.75 mg/ml in 0.9% saline which has been acidified with 1M aqueous HCl (5 µl), giving a clear solution of pH 5.0. To the solution is added 1M aqueous NaOH (2µl) to increase the pH to 6.0, resulting in a very fine suspension. The suspension is administered to the rats directly into the trachea via the mouth and vocal chords from a 1.0 ml plastic syringe and Penn-Century microsprayer. Each animal receives a 2.1 µmol/kg dose in 200 µl of suspension.

The following surgical procedure is used to prepare the animals for the experiment: the animals are anaesthetised, initially with 4% halothane, maintained with 2% halothane, both in a 1:1 oxygen: nitrous oxide mixture carrier. The anaesthetised animals are shaved on their ventral neck and craniodorsal back, then swabbed with a solution of 5% Hibitane in a 70% solution of ethanol in water. A 10 mm longitudinal incision is made in the ventral neck, the connective tissue, salivary glands, omohyoideus and sternohyoideus muscle are separated by blunt dissection and the left common carotid artery is exposed, cleared of the Vagus nerve and then elevated. The carotid artery is clipped and cannulated with a 150 mm length of pp50 Portex tubing attached to a 1 ml syringe with a 26G blunt needle filled with heparinised saline (500 i.u./ml in 0.9% saline). 10 mm of cannula is inserted, tied in place using braided silk sutures proximal and distal to the insertion point, and the clip is removed. A 2 mm incision and subcutaneous tunnel is made in the craniodorsal neck between the scapula with a 100 mm 16G curved gavage needle emerging adjacent to the cannulation site and right salivary gland. The cannula is clamped 20 mm above the insertion point with artery forceps covered with polythene tubing to avoid damaging the cannula. The syringe and needle are removed from the cannula and the free end passed through the gavage needle. The gavage needle is withdrawn and the syringe and needle are replaced on the cannula. The forceps are removed and the cannula is further withdrawn to form a gentle curve across the trachea, allowing the incision to be closed without interference, enabling free blood flow to be tested by drawing back the syringe. 50 µl of heparinised saline is administered. The ventral incision is closed with Michel clips (12 mm x 2.5 mm). A small suture loop is formed 10 mm distal to the cannula exit point and the exteriorised cannula tied to it so that it lies along the midline of the back. The cannula is again tested for free blood flow and a further 50 µl of heparinised saline is administered. The cannula is clamped with modified artery forceps near to the securing suture and cut to a length of 20 mm. A dressmakers pin is inserted to seal the cannula and the forceps are removed. Buprenorphine (Vetergesic, Reckitt and Colman) is administered (0.06 mg/kg i.m.) and the animals are placed in cages over a heated pad until fully ambulatory. Thereafter, they are placed in metabolism cages with free access to food and water. The patency of the cannula is assessed immediately before experimentation.

At 0 min, i.e. prior to dosing with Compound A, a 300 µl sample of blood is collected from the cannula into a 2.0 ml plastic syringe containing 60 µl aqueous 3.8% tri-sodium citrate. Samples are immediately transferred to a 1.9 ml Eppendorf tube and held on ice. Serial blood samples are withdrawn at ten time intervals up to 1440 minutes after dosing with Compound A. At each time point, the withdrawn blood sample is replaced with an equal volume of heparinised saline (10 i.u./ml in 0.9% saline). After the final blood sample, the rats are killed by an overdose of sodium pentobarbitone (Sagatal, Rhone Merieux, UK), followed by cervical dislocation.

### Withdrawn blood samples are analysed as follows:

Chilled whole blood samples are centrifuged at 11,000 rpm (Hereaus Biofuge 15) for 6 minutes and the plasma supernatant collected. The plasma (100 µl) is placed into an extraction tube containing acetonitrile (300 µl). After shaking for 10 minutes and centrifuging for 12 minutes, 200 µl of supernatant is transferred from the extraction tube to an autosampler vial and diluted with water (200 µl). The resulting sample is analysed by LC-MS/MS (liquid chromatography - tandem mass spectrometry). Calibration samples are prepared by adding 1 µl of a 10 mM solution of Compound A in dimethyl sulfoxide to 2 ml of blank rat plasma to give a 5 µM plasma standard, and diluting this plasma standard with blank plasma to give final concentrations of 1.67 µM, 0.56 µM, 0.19 µM, 0.062 µM, 0.021 µM and 0.007 µM. Validation samples are prepared by appropriate dilution of the 5 µM plasma standard with blank rat plasma to give concentrations of 1, 0.5, 0.2, 0.1 and 0.05 µM. The calibration standard samples are analysed by LC-MS/MS as for the withdrawn plasma samples. Calibration curves (y = mx + b), represented by the plots of the peak area ratios (y) of Compound A versus the concentrations (x) of the calibration samples, are generated using weighted (1/x) linear least-squares regression as the mathematical model. Concentrations in the withdrawn, calibration and validation samples are calculated from the resulting peak areas and the regression equation of the calibration curve. Pharmacokinetic parameters are calculated by Win Nonlin (Professional version 1.5) using non-compartmental analysis. The results (mean for 6 animals) give a Tmax of 5 minutes, i.e. the concentration of Compound A in plasma reaches a maximum within 5 minutes after intratracheal administration.

### Example 94

The absorption time Tmax for Compound A, following administration as a dry powder (particle size 0-5 µM) is measured in a rodent pharmacokinetic model which simulates inhalation as a route of administration essentially as described in Example 93.

In this case compound A (citrate salt) is formulated as a dry powder by micronisation to give an average particle diameter of less than 5 µM and then carefully diluted in lactose to give a mixture which contains Compound A diluted by weight to 40%. A calculated nominal dose of 8.4 µmol/kg is administered to each of 3 male Wistar:Han rats by administration directly into the trachea via the mouth and vocal chords from a Penn-Century dry powder delivery device actuated via a 2ml syringe containing air as a propellent.

The time to peak plasma concentration is determined as 2 minutes.

### Example 95

The absorption time Tmax for 1-cyclopentyl-3-ethyl-6-(3-ethoxy-4-pyridyl)-pyrazolo[3,4-d]pyrimidin-4-one, hereinafter referred to as Compound C, following administration by dry powder inhalation is measured in a rodent pharmacokinetic model which simulates inhalation as a route of administration essentially as described for Compound A in Example 93 with the following significant variations:

Compound C is formulated as a dry powder to give a 6% blend with lactose.

A calculated nominal dose of 2.1 µmol/kg is administered to each of 4 male Wistar:Han rats by administration directly into the trachea via the mouth and vocal chords from a Penn-Century dry powder delivery device actuated via a 2ml syringe containing air as a propellent.

The absorption time T max is determined as 2 to 20 minutes.

### Example 96

The absorption time Tmax for 1,3-dimethyl-6-(2-propoxy-5-methanesulfonylamidophenyl)-1,5-dihydropyrazolo[3,4-d]pyrimidin-4-one hereinafter referred to as Compound D, following administration by dry powder inhalation is measured in a rodent pharmacokinetic model which simulates inhalation as a route of administration essentially as described for Compound C in Example 95 with the following significant variations:

Compound D is formulated as a dry powder to give a 6% blend with lactose.

Each of 3 animals receives a dose equivalent to 2.1 µmol/kg.

The absorption time for Compound D, T max, is determined as 10 minutes.

## Claims

1. The use of a cGMP PDE 5 inhibitor in inhalable form for the preparation of an inhalable medicament for the treatment of sexual dysfunction, in which said inhibitor is 5-[2-ethoxy-5-(4-methylpiperazinyl-sulfonyl)phenyl]-1-methyl-3-n-propyl-1,6-dihydro-7H-pyrazolo[4,3-d]-pyrimidin-7-one, 4-phenylmethylamino-6-chloro-2-(1-imidazolyl)quinazoline, 4-phenyl-methylamino-6-chloro-2-(3-pyridyl)-quinazoline, 1,3-dimethyl-6-(2-propoxy-5-methane-sulfonylamidophenyl)-1,5-dihydro-pyrazolo[3,4-d]pyrimidin-4-one or 1-cyclopentyl-3-ethyl-6-(3-ethoxy-4-pyridyl)-pyrazolo[3,4-d]pyrimidin-4-one.

2. Use according to claim 1, in which the inhalable form of said medicament is an atomisable composition or a finely divided particulate form.

3. Use according to claim 1, in which said inhalable form is an aerosol comprising said inhibitor in solution or dispersion in a propellant or a nebulizable composition comprising a dispersion of said inhibitor in an aqueous or aqueous/organic medium.

4. Use according to claim 1, in which said inhalable form is a finely divided particulate form comprising said inhibitor in finely divided particulate form, optionally together with a particulate carrier.

5. A medicament comprising 5-[2-ethoxy-5-(4-methylpiperazinyl-suffonyl)phenyl]-1-methyl-3-n-propyl-1,6-dihydro-7H-pyrazolo[4,3-d]-pyrimidin-7-one, 4-phenyl-merhylamino-6-chloro-2-(1-imidazolyl)quinazoline, 4-phenyl-methylamino-6-chloro-2-(3-pyridyl)-quinazoline, 1,3-dimethyl-6-(2-propoxy-5-methane-sulfonylamidophenyl)-1,5-dihydro-pyrazolo[3,4-d]pyrimidin-4-one or 1-cyclopentyl-3-ethyl-6-(3-ethoxy-4-pyridyl)-pyrazolo[3,4-d]pyrimidin-4-one in inhalable form.

6. A medicament according to claim 5, in which the inhalable form is as specified in any one of claims 2 to 4.

7. A pharmaceutical product comprising a medicament as specified in claim 5 or 6 in association with an inhalation device.

8. An inhalation device containing a medicament as specified in claim 5 or 6.

## Patentansprüche

1. Verwendung eines cGMP-PDE 5-Inhibitors in inhalierbarer Form zur Herstellung eines inhalierbaren Arzneimittels für die Behandlung sexueller Dysfunktion, bei welcher der Inhibitor für 5-[2-Ethoxy-5-(4-methylpiperazinylsuffonyl)phenyl]-1-methyl-3-n-propyl-1,6-dihydro-7H-pyrazolo[4,3-d]pyrimidin-7-on, 4-Phenylmethylamino-6-chlor-2-(1-imidazolyl)chinazolin, 4-Phenylmethylamino-6-chlor-2-(3-pyridyl)chinazolin, 1,3-Dimethyl-6-(2-propoxy-5-methansulfonylamidophenyl)-1,5-dihydropyrazolo[3,4-d]pyrimidin-4-on oder 1-Cyclopentyl-3-ethyl-6-(3-ethoxy-4-pyridyl)pyrazolo[3,4-d]pyrimidin-4-on steht.

2. Verwendung nach Anspruch 1, bei der die inhalierbare Form des Arzneimittels für eine atomisierbare Zusammensetzung oder eine fein verteilte partikuläre Form steht.

3. Verwendung nach Anspruch 1, bei der die inhalierbare Form für ein Aerosol steht, das den Inhibitor umfasst in Lösung oder Dispersion in einem Treibmittel oder einer zerstäubbaren Zusammensetzung, die eine Dispersion des Inhibitors in einem wässrigen oder wässrig/organischen Medium umfasst.

4. Verwendung nach Anspruch 1, bei der die inhalierbare Form für eine fein verteilte partikuläre Form steht, die den Inhibitor in fein verteilter partikulärer Form, gegebenenfalls zusammen mit einem partikulären Träger, umfasst.

5. Arzneimittel, umfassend 5-[2-Ethoxy-5-(4-methylpiperazinylsulfonyl)phenyl]-1-methyl-3-n-propyl-1,6-dihydro-7H-pyrazolo[4,3-d]pyrimidin-7-on, 4-Phenylmethylamino-6-chlor-2-(1-imidazolyl)chinazolin, 4-Phenylmethylamino-6-chlor-2-(3-pyridyl)chinazotin, 1,3-Dimethyl-6-(2-propoxy-5-methansulfonylamidophenyl)-1,5-dihydropyrazolo[3,4-d]pyrimidin-4-on oder 1-Cyclopentyl-3-ethyl-6-(3-ethoxy-4-pyridyl)pyrazolo[3,4-d]pyrimidin-4-on in inhalierbarer Form.

6. Arzneimittel nach Anspruch 5, in dem die inhalierbare Form definiert ist wie in einem der Ansprüche 2 bis 4.

7. Pharmazeutisches Produkt, umfassend ein Arzneimittel, das wie in Anspruch 5 oder 6 definiert ist, in Verbindung mit einer Inhalationsvorrichtung.

8. lnhalationsvorrichtung, enthaltend ein Arzneimittel, das wie in Anspruch 5 oder 6 definiert ist.

## Revendications

1. Utilisation d'un inhibiteur de PDE 5 de GMPc sous forme inhalable pour la préparation d'un médicament inhalable pour le traitement d'un dysfonctionnement sexuel, dans laquelle ledit inhibiteur est la 5-[2-éthoxy-5-(4-méthylpipérazinyl-sulfonyl)-phényl]-1-méthyl-3-n-propyl-1,6-dihydro-7H-pyrazolo[4,3-d]-pyrimidin-7-one, la 4-phénylméthylamino-6-chloro-2-(1-imidazolyl)-quinazoline, la 4-phénylméthylamino-6-chloro-2-(3-pyridyl)-quinazoline, la 1,3-diméthyl-6-(2-propoxy-5-méthane-sulfonylamido-phényl)-1,5-dihydro-pyrazolo[3,4-d]pyrimidin-4-one ou la 1-cyclopentyl-3-éthyl-6-(3-éthoxy-4-pyridyl)-pyrazolo[3,4-d]pyrimidin-4-one.

2. Utilisation selon la revendication 1, dans laquelle la forme inhalable dudit médicament est une composition atomisable ou une forme particulaire finement divisée.

3. Utilisation selon la revendication 1, dans laquelle ladite forme inhalable est un aérosol comprenant ledit inhibiteur en solution ou en dispersion dans un gaz propulseur, ou une composition nébulisable comprenant une dispersion dudit inhibiteur dans un milieu aqueux ou aqueux/organique.

4. Utilisation selon la revendication 1, dans laquelle ladite forme inhalable est une forme particulaire finement divisée comprenant ledit inhibiteur sous forme particulaire finement divisée, facultativement conjointement à un support particulaire.

5. Médicament comprenant la 5-[2-éthoxy-5-(4-méthylpipérazinyl-sulfonyl)-phényl]-1-méthyl-3-n-propyl-1,6-dihydro-7H-pyrazolo[4,3-d]-pyrimidin-7-one, la 4-phénylméthylamino-6-chloro-2-(1-imidazolyl)-quinazoline, la 4-phénylméthylamino-6-chloro-2-(3-pyridyl)-quinazoline, la 1,3-diméthyl-6-(2-propoxy-5-méthane-sulfonylamidophényl)-1,5-dihydropyrazolo[3,4-d]pyrimidin-4-one ou la 1-cyclopentyl-3-éthyl-6-(3-éthoxy-4-pyridyl)-pyrazolo[3,4-d]pyrimidin-4-one sous forme inhalable.

6. Médicament selon la revendication 5, dans lequel la forme inhalable est telle que spécifiée dans l'une quelconque des revendications 2 à 4.

7. Produit pharmaceutique comprenant un médicament tel que spécifié dans la revendication 5 ou 6, en association avec un dispositif d'inhalation.

8. Dispositif d'inhalation contenant un médicament tel que spécifié dans la revendication 5 ou 6.
